(19) Europäisches Patentamt
European Patent Office
Office européen des brevets

(11) **EP 4 156 193 B1**

(12) **EUROPEAN PATENT SPECIFICATION**

(45) Date of publication and mention
of the grant of the patent:
**12.11.2025 Bulletin 2025/46**

(21) Application number: **22174824.7**

(22) Date of filing: **23.05.2022**

(51) International Patent Classification (IPC):
**G16B 15/20** (2019.01)     **G16B 15/30** (2019.01)

(52) Cooperative Patent Classification (CPC):
**G16B 15/20; G16B 15/30**

(54) **APPARATUS, METHOD AND PROGRAM FOR GENERATING AN INITIAL CONFORMATION**

VORRICHTUNG, VERFAHREN UND PROGRAMM ZUR ERZEUGUNG EINER AUSGANGSKONFORMATION

APPAREIL, PROCÉDÉ ET PROGRAMME DE GÉNÉRATION DE CONFORMATION INITIALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.09.2021 JP 2021156817**

(43) Date of publication of application:
**29.03.2023 Bulletin 2023/13**

(73) Proprietor: **FUJITSU LIMITED**
**Kawasaki-shi, Kanagawa 211-8588 (JP)**

(72) Inventor: **Tanida, Yoshiaki**
**Kawasaki-shi, Kanagawa 211-8588 (JP)**

(74) Representative: **Hoffmann Eitle**
**Patent- und Rechtsanwälte PartmbB**
**Arabellastraße 30**
**81925 München (DE)**

(56) References cited:
US-A- 5 241 470          US-A1- 2003 135 331
US-A1- 2018 260 517      US-A1- 2020 176 074

• OLENDOR R ET AL: "A fast algorithm for searching for molecules containing a pharmacophore in very large virtual combinatorial libraries", JOURNAL OF CHEMICAL INFORMATION AND COMPUTER SCIENCES, AMERICAN CHEMICAL SOCIETY, COLOMBUS,OHIO, US, vol. 41, no. 3, 29 May 2001 (2001-05-29), pages 731 - 738, XP001058995, ISSN: 0095-2338, DOI: 10.1021/CI000463O
• MCHUGH SEAN M ET AL: "Computational methods to design cyclic peptides", CURRENT OPINION IN CHEMICAL BIOLOGY, CURRENT BIOLOGY LTD, LONDON, GB, vol. 34, 1 September 2016 (2016-09-01), pages 95 - 102, XP029806927, ISSN: 1367-5931, DOI: 10.1016/J.CBPA.2016.08.004
• KATAOKA T ET AL: "The utility of side-chain cyclization in determining the receptor-bound conformation of peptides: cyclic tripeptides and angiotensin II.", BIOPOLYMERS, JOHN WILEY, HOBOKEN, USA, vol. 32, no. 11, 1 November 1992 (1992-11-01), pages 1519 - 1533, XP001062669, ISSN: 0006-3525, DOI: 10.1002/BIP.360321110

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD

The present disclosure relates to an initial conformation generation apparatus, an initial conformation generation method, and an initial conformation generation program.

BACKGROUND

In recent years, in the field of drug discovery, drug discovery based on middle molecules (molecular weight of 500 to 3000) supposed to cause less side effects has been expected, and development of a search method for searching for a stable conformation of a middle molecule has been in progress.

For example, a cyclic peptide molecule has been drawing great attention because cyclization greatly reduces the entropy, which makes it possible to generate a compound having great binding activity.

Japanese Laid-open Patent Publication Nos. 2010-159218 and 2020-091518 and U.S. Patent Application Publication No. 2018/0260517 are disclosed as related art.

Also, F. Jiang and H. Geng, "Computational Methods for Studying Conformational Behaviors of Cyclic Peptides", 2019 is disclosed as the related art.

Finally, R. Olender and R. Rosenfeld, "A fast algorithm for searching for molecules containing a pharmacophore in very large virtual combinatorial libraries", 2001 relates to an algorithm for identifying molecules displaying a particular pharmacophore, based on the construction and screening of supermolecules using predefined sets of chemical scaffolds.

SUMMARY

[TECHNICAL PROBLEM]

In searching for a stable conformation, it is important to search an appropriate search range, and whether the search range is optimized or not largely depends on an initial conformation.

Meanwhile, in the case of a cyclic peptide molecule, the initial conformation has been generated by a method of cyclizing a linear peptide molecule by linking a head and a tail thereof. For this reason, in the generated initial conformation, each of amino acid residue sequences constituting the cyclic peptide molecule has such low degrees of freedom that an appropriate search range may not be searched in searching for a stable conformation.

According to one aspect, an object is to optimize a search range in searching for a stable conformation of a cyclic peptide molecule.

[ADVANTAGEOUS EFFECTS OF INVENTION]

A search range in searching for a stable conformation of a cyclic peptide molecule may be optimized.

[SOLUTION TO PROBLEM]

The present invention is defined by the independent claims. Preferred embodiments are defined by the dependent claims. Further aspects are provided for facilitating the understanding of the invention.

According to an aspect of the embodiments, an initial conformation generation apparatus includes a generation unit that generates a model representing a cyclic peptide molecule by identifying $C\alpha$ atoms of each of a plurality of amino acid residues, by arranging the identified $C\alpha$ atoms on a circumference, and by adding main chains and side chains of the plurality of amino acid residues; and a search instruction unit that causes a search unit to search for a stable conformation of the cyclic peptide molecule by using the generated model as an initial conformation of the cyclic peptide molecule.

BRIEF DESCRIPTION OF DRAWINGS

FIG. 1 is a diagram illustrating an example of a system configuration of a stable conformation search system and functional configurations of a terminal apparatus and a server apparatus;
FIG. 2A and FIG. 2B are diagrams illustrating an example of hardware configurations of the terminal apparatus and the server apparatus;
FIG. 3 is a diagram illustrating an example of a functional configuration of a cyclic peptide molecule generation unit;

FIG. 4 is a diagram illustrating a specific example of processing by a Cα atom arrangement unit;

FIG. 5 is a diagram illustrating a specific example of processing by an addition unit and a conformational relaxation unit;

FIG. 6 is a diagram for explaining backbone dihedral angles;

FIG. 7 is a first diagram illustrating an example of a dihedral angle distribution diagram;

FIG. 8 is a second diagram illustrating an example of a dihedral angle distribution diagram; and

FIG. 9 is a flowchart illustrating a sequence of stable conformation search processing.

DESCRIPTION OF EMBODIMENTS

Hereinafter, the embodiments will be described with reference to the accompanying drawings. In the present specification and drawings, constituent elements having substantially the same functional configuration will be denoted with the same reference sign, whereby repetitive description thereof will be omitted.

[First Embodiment]

<System Configuration of Stable Conformation Search System and Functional Configurations of Terminal Apparatus and Server Apparatus>

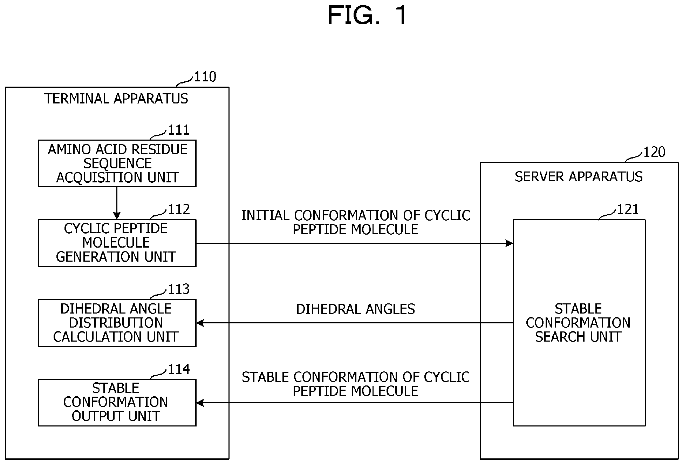

First, description will be given of a system configuration of a stable conformation search system according to a first embodiment and functional configurations of a terminal apparatus and a server apparatus constituting the stable conformation search system. FIG. 1 is a diagram illustrating an example of the system configuration of the stable conformation search system and the functional configurations of the terminal apparatus and the server apparatus.

A stable conformation search system 100 is a system for searching for a stable conformation of a middle molecule and, for example, a system for searching for a stable conformation of a cyclic peptide molecule having multiple amino acid residue sequences.

As illustrated in FIG. 1, the stable conformation search system 100 includes a terminal apparatus 110, which is an example of an initial conformation generation apparatus, and a server apparatus 120.

A search program is installed in the terminal apparatus 110. Executing the program, the terminal apparatus 110 functions as an amino acid residue sequence acquisition unit 111, a cyclic peptide molecule generation unit 112, a dihedral angle distribution calculation unit 113, and a stable conformation output unit 114.

The amino acid residue sequence acquisition unit 111 is an example of an acquisition unit, and acquires multiple amino acid residue sequences constituting a cyclic peptide molecule of a search target when a user of the stable conformation search system 100 inputs these amino acid residue sequences.

When the user of the stable conformation search system 100 inputs a parameter (a distance "a" to be described later) for use to generate an initial conformation of the cyclic peptide molecule of the search target, the amino acid residue sequence acquisition unit 111 acquires the parameter.

The cyclic peptide molecule generation unit 112 is an example of a generation unit, and generates, as the initial conformation of the cyclic peptide molecule, a model representing the cyclic peptide molecule under the acquired parameter based on the multiple amino acid residue sequences acquired by the amino acid residue sequence acquisition unit 111. The cyclic peptide molecule generation unit 112 is also an example of a search instruction unit, and transmits information indicating the generated initial conformation of the cyclic peptide molecule to the server apparatus 120, thereby instructing the server apparatus 120 to search for a stable conformation based on the initial conformation. Details of a method for generating the initial conformation of the cyclic peptide molecule by the cyclic peptide molecule generation unit 112 will be described later.

The dihedral angle distribution calculation unit 113 is an example of a calculation unit. In response to transmission of the information indicating the initial conformation of the cyclic peptide molecule by the cyclic peptide molecule generation unit 112, the dihedral angle distribution calculation unit 113 acquires information indicating backbone dihedral angles calculated in the process of searching for the stable conformation from the server apparatus 120. The dihedral angle distribution calculation unit 113 calculates a dihedral angle distribution based on the acquired information indicating the backbone dihedral angles, and outputs a dihedral angle distribution diagram.

In response to the transmission of the information indicating the initial conformation of the cyclic peptide molecule by the cyclic peptide molecule generation unit 112, the stable conformation output unit 114 acquires information indicating the searched-out stable conformation of the cyclic peptide molecule from the server apparatus 120. The stable conformation output unit 114 outputs the acquired information indicating the stable conformation of the cyclic peptide molecule.

The server apparatus 120 functions as a stable conformation search unit 121. The stable conformation search unit 121 is an example of a search unit. When receiving the information indicating the initial conformation of the cyclic peptide molecule from the terminal apparatus 110, the stable conformation search unit 121 searches for a stable conformation of the cyclic peptide molecule based on the received information. The stable conformation search unit 121 transmits information indicating the backbone dihedral angles calculated in the process of searching for the stable conformation, to the terminal apparatus 110. The stable conformation search unit 121 transmits the information indicating the searched-out stable conformation of the cyclic peptide molecule to the terminal apparatus 110.

Based on the information indicating the initial conformation of the cyclic peptide molecule transmitted from the terminal apparatus 110, the stable conformation search unit 121 searches for the stable conformation by, for example, a generalized-ensemble method. Examples of the generalized-ensemble method mentioned herein include a multi-canonical method, a simulated tempering method, a replica exchange method (for example, replica exchange with solute tempering (REST) 2), and so on.

<Hardware Configurations of Terminal Apparatus and Server Apparatus>

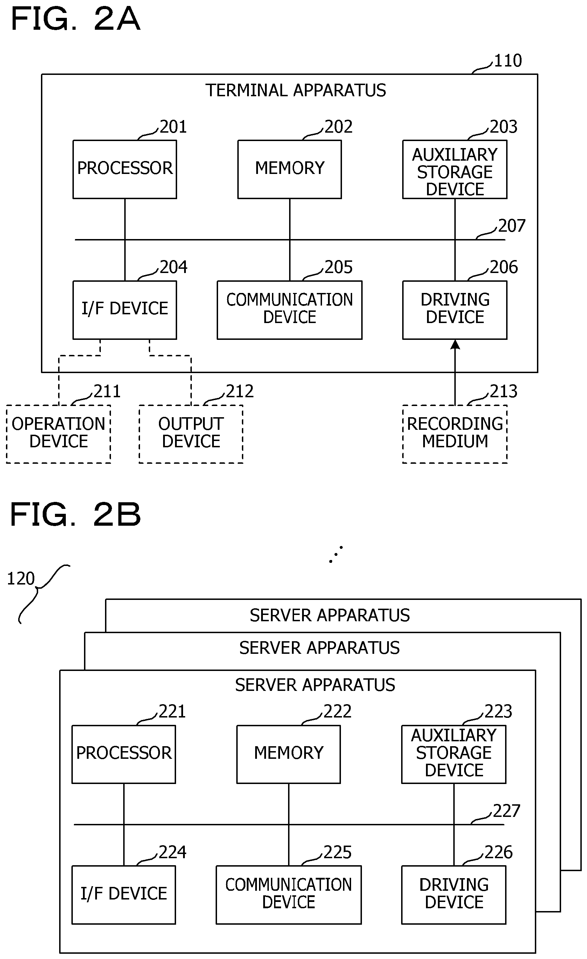

Next, hardware configurations of the terminal apparatus 110 and the server apparatus 120 will be described next. FIG. 2A and FIG. 2B are diagrams illustrating an example of the hardware configurations of the terminal apparatus and the server apparatus.

(1) Hardware Configuration of Terminal Apparatus

As illustrated in FIG. 2A, the terminal apparatus 110 includes a processor 201, a memory 202, an auxiliary storage device 203, an interface (I/F) device 204, a communication device 205, and a driving device 206. These hardware devices in the terminal apparatus 110 are coupled to each other via a bus 207.

The processor 201 includes various computing devices such as a central processing unit (CPU) and a graphics processing unit (GPU). The processor 201 loads various programs (such, for example, as a search program) onto the memory 202 and executes the programs.

The memory 202 includes main storage devices such as a read-only memory (ROM) and a random-access memory (RAM). The processor 201 and the memory 202 form a so-called computer. The computer implements the above various functions by the processor 201 executing the various programs loaded onto the memory 202.

The auxiliary storage device 203 stores the various programs and various kinds of information to be used in execution of the various programs by the processor 201.

The I/F device 204 is a coupling device that couples the terminal apparatus 110 to an operation device 211 and an output device 212, which are examples of external devices.

The communication device 205 is a communication device for communicating with the server apparatus 120 via a network.

The driving device 206 is a device in which a recording medium 213 is to be placed. Examples of the recording medium 213 mentioned herein include a medium on which information is recorded optically, electrically, or magnetically, such as a compact disc read-only memory (CD-ROM), a flexible disk, and a magneto-optical disk. The examples of the recording medium 213 may also include a semiconductor memory and the like on which information is recorded electrically, such as a ROM and a flash memory.

The various programs to be installed onto the auxiliary storage device 203 are installed, for example, in such a way that the distributed recording medium 213 is placed in the driving device 206 and the driving device 206 reads out the various programs recorded on the recording medium 213. Alternatively, the various programs to be installed onto the auxiliary storage device 203 may be installed by being downloaded from the network via the communication device 205.

(2) Hardware Configuration of Server Apparatus

As illustrated in FIG. 2B, multiple server apparatuses 120 constitute a cluster, and each server apparatus includes a processor 221, a memory 222, an auxiliary storage device 223, an interface (I/F) device 224, a communication device 225, and a driving device 226.

The hardware devices included in the server apparatuses are the same as or similar to the hardware devices included in the terminal apparatus 110, and thus description thereof is omitted herein.

<Functional Configuration of Cyclic Peptide Molecule Generation Unit>

Next, a functional configuration of the cyclic peptide molecule generation unit 112 will be described in detail. As described above, executing the search program, the terminal apparatus 110 implements the various functions of the terminal apparatus 110. Among them, the cyclic peptide molecule generation unit 112 is implemented by executing, for example, an initial conformation generation program which is a part of the search program.

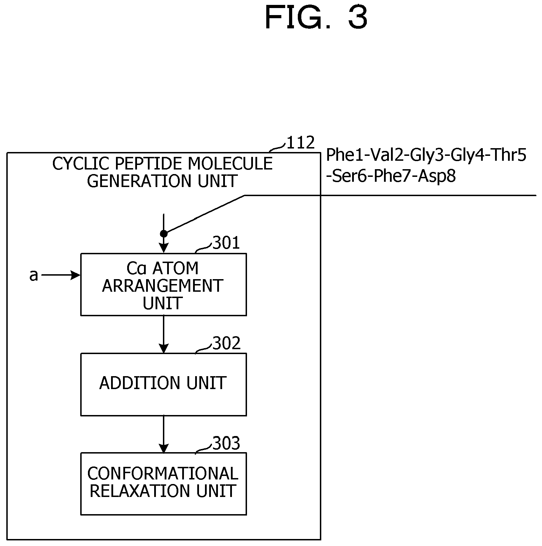

FIG. 3 is a diagram illustrating an example of the functional configuration of the cyclic peptide molecule generation unit. As illustrated in FIG. 3, the cyclic peptide molecule generation unit 112 includes a Cα atom arrangement unit 301, an addition unit 302, and a conformational relaxation unit 303.

The Cα atom arrangement unit 301 identifies a Cα atom that is an atom representing an amino acid in each of the acquired multiple amino acid residue sequences, and arranges the identified multiple Cα atoms at equal intervals on a circumference in the same plane. FIG. 3 illustrates the example in which 8 amino acid residue sequences (Phe1, Val2, Gly3, Thr5, Ser6, Phe7, and Asp8) are acquired and input to the Cα atom arrangement unit 301. The Cα atoms are arranged at equal intervals on the circumference in the same plane in order to constitute a conformation in which the multiple amino acid residue sequences (here, the 8 amino acid residue sequences) have higher degrees of freedom from each other.

When receiving a distance "a" (for example, 3.84 angstrom) between adjacent Cα atoms input by the user, the Cα atom arrangement unit 301 calculates a radius of a circumference where to arrange the Cα atoms based on the number "n" of the Cα atoms identified and the input distance "a" between the Cα atoms.

The Cα atom arrangement unit 301 arranges the Cα atoms at equal intervals along the circumference defined by the calculated radius such that the distance between the adjacent Cα atoms is "a". The equal intervals mentioned herein do not have to be exactly at the same distance, but are synonymous with approximately equal intervals.

The addition unit 302 adds the main chain and side chains of each of the amino acid residue sequences to the corresponding one of the Cα atoms arranged on the circumference in the same plane.

The conformational relaxation unit 303 sequentially performs conformational relaxation of each of the amino acid residue sequences generated by adding the main chain and the side chains of each of the amino acid residue sequence to the corresponding one of the Cα atoms, thereby generating a model representing the cyclic peptide molecule having the multiple amino acid residue sequences as an initial conformation of the cyclic peptide molecule.

<Specific Example of Processing by Cα Atom Arrangement Unit>

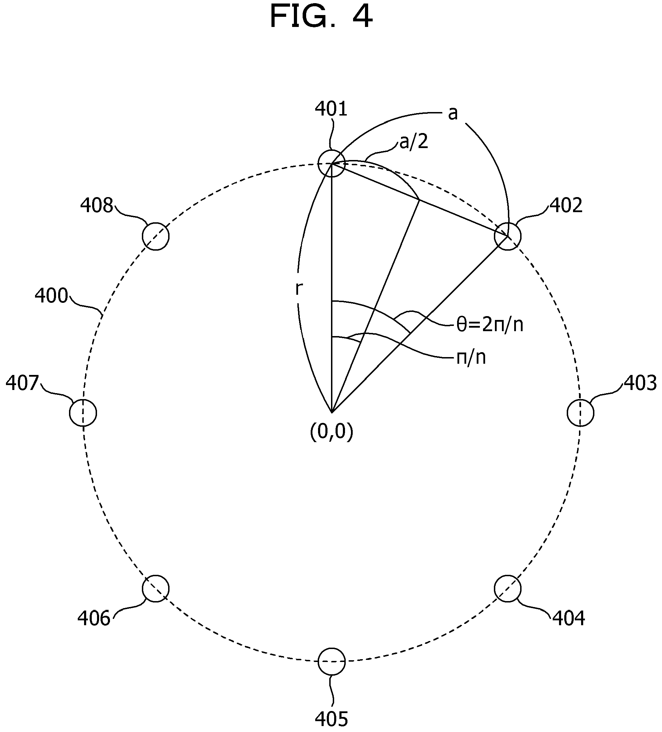

Next, a specific example of processing by the Cα atom arrangement unit 301 will be described. FIG. 4 is a diagram illustrating the specific example of the processing by the Cα atom arrangement unit. FIG. 4 illustrates the example in which Cα atoms 401 to 408 are identified respectively in n = 8 amino acid residue sequences and arranged at equal intervals on a circumference 400 in the same plane.

As illustrated in FIG. 4, in the case of the 8 Cα atoms, among straight lines extending from the center coordinates (0, 0) of the circumference 400 to the respective Cα atoms, the straight lines extending to adjacent Cα atoms form an angle θ of 2π/n (= π/4).

As illustrated in FIG. 4, letting "a" be the distance between adjacent Cα atoms, a radius r of the circumference 400 may be calculated in accordance with the following formula (1):

$$\text{(Formula 1)} \quad r = (a/2) \times [\sin(\pi/n)]^{-1}.$$

The coordinates of each of the Cα atoms 401 to 408 may be calculated in accordance with the following formula (2):

$$\text{(Formula 2)} \quad \text{Coordinates} = [r\sin(n-1)\,\theta_x,\ r\cos(n-1)\,\theta_x].$$

In the above formula (2), r denotes the radius of the circumference 400 calculated by the formula (1), and $\theta_x$ may be calculated in accordance with the following formula (3):

$$\text{(Formula 3)} \quad \theta_x = x \times 2\pi/n,$$

where x is any integer of 1 to n.

<Specific Example of Processing by Addition Unit and Conformational Relaxation Unit>

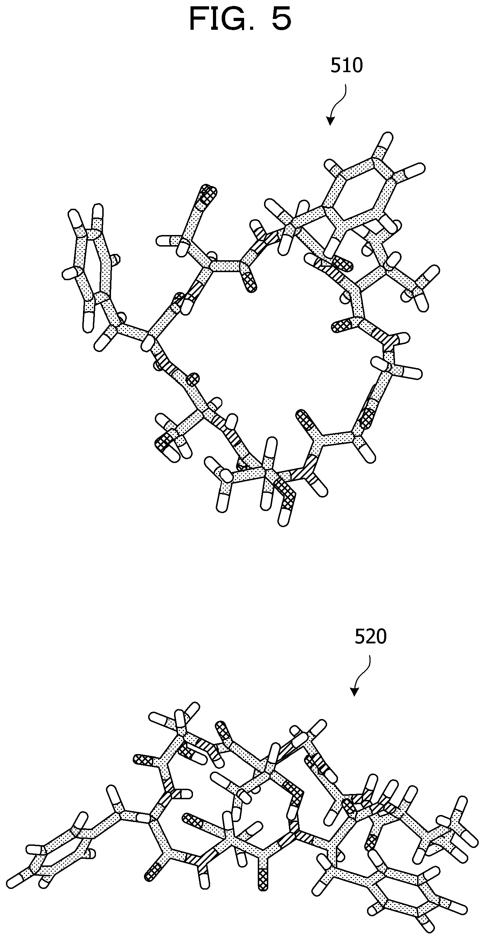

Next, a specific example of processing by the addition unit 302 and the conformational relaxation unit 303 will be described. FIG. 5 is a diagram illustrating the specific example of the processing by the addition unit and the conformational relaxation unit.

In FIG. 5, reference sign 510 illustrates an addition result in which the addition unit 302 adds the main chain and the side chains of each of the amino acid residue sequences to the corresponding one of the Cα atoms 401 to 408 arranged at the equal intervals on the circumference in the same plane by the Cα atom arrangement unit 301. When acquiring the addition result illustrated with reference sign 510 from the addition unit 302, the conformational relaxation unit 303 relaxes the post-addition conformation so as to reduce the entropy, and thereby generates a model representing the cyclic peptide molecule as an initial conformation of the cyclic peptide molecule.

Reference sign 520 illustrates a case where the initial conformation of the cyclic peptide molecule is generated in accordance with the method in the related art. The method in the related art is a method in which a linear peptide molecule is generated by linking 8 amino acid residue sequences in a linear chain form, and then cyclized by linking the head and the tail of the generated linear peptide molecule.

As is apparent from the comparison between reference signs 510 and 520, the initial conformation illustrated with reference sign 510 is a conformation in which the 8 amino acid residue sequences are farther from each other and therefore have higher degrees of freedom than in the initial conformation illustrated with reference sign 520.

<Specific Example of Processing by Dihedral Angle Distribution Calculation Unit>

Next, a specific example of processing by the dihedral angle distribution calculation unit 113 will be described. As described above, the dihedral angle distribution calculation unit 113 acquires the information indicating the backbone dihedral angles calculated in the process of searching for the stable conformation from the server apparatus 120, calculates the dihedral angle distribution based on the acquired information indicating the dihedral angles, and outputs the dihedral angle distribution diagram.

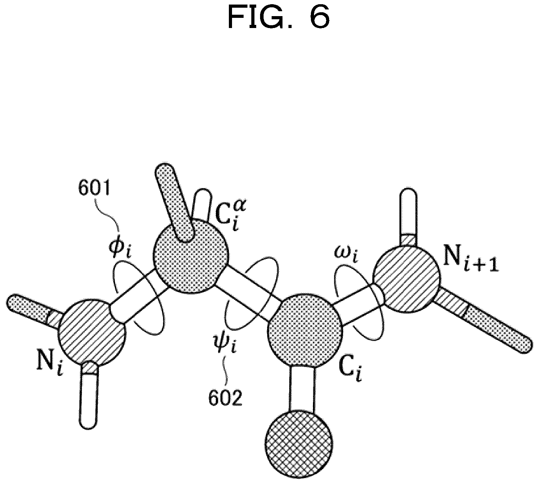

The backbone dihedral angles will be briefly described herein. FIG. 6 is a diagram for explaining the backbones dihedral angle. In the present embodiment, the dihedral angle distribution calculation unit 113 acquires $(\varphi, \Psi)$ as the information indicating the dihedral angles from the server apparatus 120. In FIG. 6, reference signs 601 and 602 respectively point to $\varphi_i$ and $\psi_i$ which are information indicating an i-th dihedral angle in the information indicating the dihedral angles acquired by the dihedral angle distribution calculation unit 113.

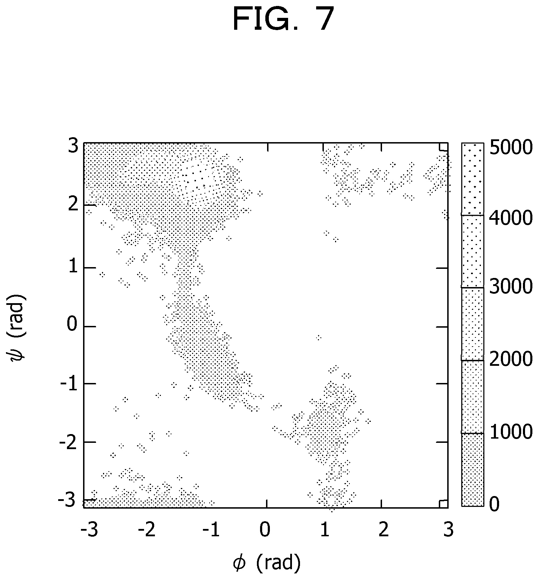

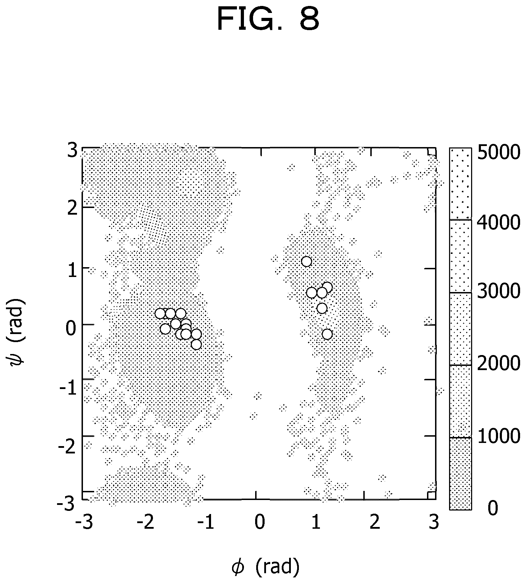

Subsequently, a dihedral angle distribution diagram will be described. FIGs. 7 and 8 are first and second diagrams illustrating examples of dihedral angle distribution diagrams. In FIGs. 7 and 8, the horizontal axis indicates $\varphi$ [rad] in the information indicating the dihedral angles, and the vertical axis indicates $\psi$ [rad] in the information indicating the dihedral angles. Each plot (Ramachandran plot) in the figures indicates that information indicating a dihedral angle corresponding to each position is calculated in the process of searching for a stable conformation, and a difference in color in the plot represents a difference in the number of searches performed on the information.

For example, the closer to red, the larger the number of searches is, and the closer to blue, the smaller the number of searches is. A white region indicates that the region is not searched even once.

FIG. 7 is a dihedral angle distribution diagram of Ser6 in a case where an initial conformation for 8 amino acid residue sequences (Phe1, Val2, Gly3, Thr5, Ser6, Phe7, and Asp8) is generated by the method in the related art and then a stable conformation is searched for. As described above, the method in the related art is the method in which a linear peptide molecule is generated by linking 8 amino acid residue sequences in a linear chain form, and is cyclized by linking the head and the tail of the generated linear peptide molecule.

FIG. 8 is a dihedral angle distribution diagram of Ser6 in a case where an initial conformation for 8 amino acid residue sequences (Phe1, Val2, Gly3, Thr5, Ser6, Phe7, and Asp8) is generated by the cyclic peptide molecule generation unit 112 and then a stable conformation is searched for.

As seen from the comparison between FIGs. 7 and 8, a difference between the initial conformations results in a large difference between the dihedral angle distribution diagrams. For example, in the search for a stable conformation by the server apparatus 120, the search range significantly differs between the different initial conformations.

In the present embodiment, which of the search ranges is more appropriate is examined by the following procedure: · acquiring 20 kinds of stable conformations obtained by a nuclear magnetic resonance experiment for Ser6 among the above 8 amino acid residue sequences (Phe1, Val2, Gly3, Thr5, Ser6, Phe7, and Asp8); · plotting, as correct answer data, the information indicating the dihedral angles in each of the 20 kinds of stable conformations acquired for Ser6 over a dihedral angle distribution diagram; and · examining whether the correct answer data is included in the search range indicated by the plot of each color in the dihedral angle distribution diagram by checking a degree of overlap between the search range and the white circles plotted as the correct answer data.

In FIG. 8, 20 white circles represent the information indicating the dihedral angles in the 20 kinds of stable conformations acquired for Ser6. As illustrated in FIG. 8, all the white circles are located on the plot of blue to light blue, and are searched at least once by the stable conformation search unit 121 in the search for the stable conformation.

When the 20 white circles illustrated in FIG. 8 are plotted in the same positions over the dihedral angle distribution diagram illustrated in FIG. 7, some of the 20 white circles are plotted in the white region. For example, when the initial conformation of the cyclic peptide molecule is generated by the method in the related art, some of the 20 kinds of stable conformations are not searched even once in the search for the stable conformation.

For example, in a case where the initial conformation of the cyclic peptide molecule is generated by operating the cyclic peptide molecule generation unit 112, it is possible to search stable conformations that are not searched in a case where the initial conformation is generated by the method in the related art. For example, in the case where the initial conformation of the cyclic peptide molecule is generated by operating the cyclic peptide molecule generation unit 112, the server apparatus 120 is enabled to search a more appropriate search range than in the case where the initial conformation is generated by the method in the related art.

<Sequence of Stable Conformation Search Processing>

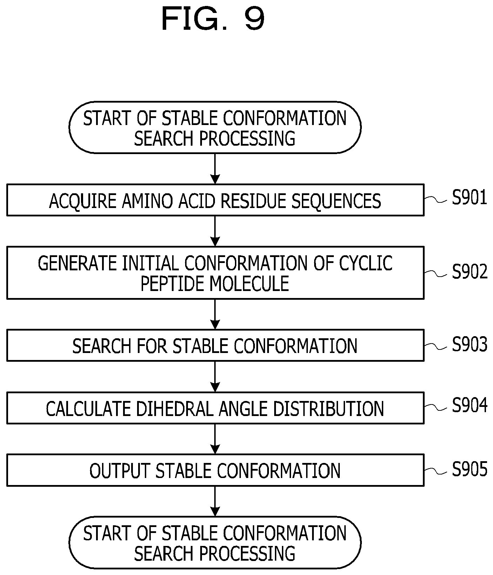

Next, a sequence of entire stable conformation search processing will be described. FIG. 9 is a flowchart illustrating the sequence of the stable conformation search processing.

At step S901, the terminal apparatus 110 acquires multiple amino acid residue sequences. The terminal apparatus 110 acquires a distance a between adjacent Cα atoms.

At step S902, the terminal apparatus 110 identifies the Cα atom of each of the acquired multiple amino acid residue sequences, and arranges the Cα atoms at equal intervals on the circumference in the same plane calculated based on the distance a. The terminal apparatus 110 adds the main chain and side chains of each of the amino acid residue sequence to the corresponding one of the arranged Cα atoms, and relaxes the post-addition conformation, thereby generating the initial conformation of the cyclic peptide molecule.

At step S903, the terminal apparatus 110 transmits information indicating the generated initial conformation of the cyclic peptide molecule to the server apparatus 120, thereby giving an instruction to search for a stable conformation of the cyclic peptide molecule.

At step S904, the terminal apparatus 110 acquires the information indicating the backbone dihedral angles calculated in the process of searching for the stable conformation from the server apparatus 120, calculates the dihedral angle distribution based on the acquired information indicating the dihedral angles, and outputs the dihedral angle distribution diagram to the user.

At step S905, the terminal apparatus 110 acquires the information indicating the searched-out stable conformation of the cyclic peptide molecule from the server apparatus 120, and outputs the information to the user.

As is clear from the above description, the terminal apparatus 110 according to the first embodiment identifies the Cα atom of each of the multiple amino acid residue sequences, arranges the Cα atoms at the equal intervals on the circumference, and adds the main chain and the side chains of each of the multiple amino acid residue sequences to the corresponding one of the Cα atoms arranged on the circumference. In this way, the terminal apparatus 110 according to the first embodiment is capable of generating the initial conformation of the cyclic peptide molecule in which each of the multiple amino acid residue sequences has higher conformational degrees of freedom.

The terminal apparatus 110 according to the first embodiment transmits the information indicating the generated initial conformation of the cyclic peptide molecule to the server apparatus 120, thereby giving the instruction to search for the stable conformation of the cyclic peptide molecule.

According to the first embodiment, it is thus possible to optimize a search range in a search for a stable conformation of a cyclic peptide molecule.

[Second Embodiment]

The first embodiment is described above for the case where the stable conformation search system 100 is constituted by the terminal apparatus 110 and the server apparatuses 120. However, the stable conformation search system 100 may be constituted by the terminal apparatus 110, the server apparatus 120, and another apparatus (for example, three or more apparatuses). Alternatively, the stable conformation search system 100 may be constituted by an apparatus in which the terminal apparatus 110 and the server apparatus 120 are integrated (for example, a single apparatus).

The first embodiment is described above for the case where the terminal apparatus 110 includes the amino acid residue sequence acquisition unit 111 to the stable conformation output unit 114, and the server apparatus 120 includes the stable conformation search unit 121. However, some functions of the terminal apparatus 110 may be implemented in the server apparatus 120. Alternatively, some functions of the server apparatus 120 may be implemented in the terminal apparatus 110.

Although the first embodiment is described above for the example in which the 8 amino acid residue sequences are used to generate the initial conformation of the cyclic peptide molecule, the number of amino acid residue sequences for generating the initial conformation of the cyclic peptide molecule may be any number. Although the first embodiment is described above for the case where the dihedral angle distribution diagram for Ser6 among the 8 amino acid residue sequences is output, the dihedral angle distribution diagrams for the remaining 7 amino acid residue sequences may be output together.

Although details of the conformational relaxation are not described in the first embodiment, the conformational relaxation unit 303 may relax the conformation while keeping each Cα atom fixed, for example. Alternatively, the conformational relaxation unit 303 may relax the conformation including each Cα atom. Instead, the search for the stable conformation may be started without the conformational relaxation by the conformational relaxation unit 303 being executed.

The present disclosure is not limited to the configurations illustrated herein but may include configurations such as a combination of any of the configurations exemplified in the aforementioned embodiments with other elements. These aspects may be changed without departing from the present disclosure and appropriately set in accordance with application modes thereof.

**Claims**

1.    An initial conformation generation apparatus comprising:

a generation unit that generates a model representing a cyclic peptide molecule by identifying Cα atoms of each of a plurality of amino acid residues, by arranging the identified Cα atoms on a circumference, and by adding main chains and side chains of the plurality of amino acid residues; and
a search instruction unit that causes a search unit to search for a stable conformation of the cyclic peptide molecule by using the generated model as an initial conformation of the cyclic peptide molecule; and
a calculation unit that calculates a dihedral angle distribution based on backbone dihedral angles calculated when the stable conformation is searched for,
wherein the search instruction unit causes the search unit to search for the stable conformation by using a search range as the initial conformation, the search range being indicated by the dihedral angle distribution, the search range including correct answer data that indicates dihedral angle of each of the plurality of amino acid residues.

2.    The initial conformation generation apparatus according to claim 1, wherein
the generation unit arranges the Cα atoms at equal intervals on the circumference.

3.    The initial conformation generation apparatus according to claim 1, wherein
the generation unit arranges each of n Cα atoms at a position of coordinates = [rsin (n-1) $\theta_x$, rcos (n-1) $\theta_x$], a distance between adjacent Cα atoms being a, r being (a/2) $\times$ [sin $(\pi/n)$]$^{-1}$, $\theta_x$ being x $\times$ 2n/n, and x being an integer of 1 to n.

4.    The initial conformation generation apparatus according to claim 1, wherein
the generation unit generates the model representing the cyclic peptide molecule by rearranging a conformation after the adding.

5.    The initial conformation generation apparatus according to claim 4, wherein
the generation unit rearranges the conformation after the adding with keeping arrangement of the Cα atoms fixed.

6.    The initial conformation generation apparatus according to claim 1, wherein
the calculation unit outputs the dihedral angle distribution in colors each corresponding to the number of searches for the stable conformation performed.

7.    The initial conformation generation apparatus according to claim 1, wherein
the search unit searches for the stable conformation by a generalized-ensemble method using the generated model as the initial conformation of the cyclic peptide molecule.

8.    An initial conformation generation method for a computer to execute a process comprising:

generating a model representing a cyclic peptide molecule by identifying Cα atoms of each of a plurality of amino acid residues, by arranging the identified Cα atoms on a circumference, and by adding main chains and side chains of the plurality of amino acid residues; and
causing a search unit to search for a stable conformation of the cyclic peptide molecule by using the generated model as an initial conformation of the cyclic peptide molecule; and
calculating a dihedral angle distribution based on backbone dihedral angles calculated when the stable conformation is searched for,
wherein the causing includes causing the search unit to search for the stable conformation by using a search range as the initial conformation, the search range being indicated by the dihedral angle distribution, the search range including correct answer data that indicates dihedral angle of each of the plurality of amino acid residues.

**9.** An initial conformation generation program that causes at least one computer to execute a process, the process comprising:

generating a model representing a cyclic peptide molecule by identifying $C\alpha$ atoms of each of a plurality of amino acid residues, by arranging the identified $C\alpha$ atoms on a circumference, and by adding main chains and side chains of the plurality of amino acid residues; and
causing a search unit to search for a stable conformation of the cyclic peptide molecule by using the generated model as an initial conformation of the cyclic peptide molecule; and
calculating a dihedral angle distribution based on backbone dihedral angles calculated when the stable conformation is searched for,
wherein the causing includes causing the search unit to search for the stable conformation by using a search range as the initial conformation, the search range being indicated by the dihedral angle distribution, the search range including correct answer data that indicates dihedral angle of each of the plurality of amino acid residues.

**Patentansprüche**

**1.** Einrichtung zur Erzeugung einer Ausgangskonformation, umfassend:

eine Erzeugungseinheit, die ein Modell erzeugt, das ein zyklisches Peptidmolekül darstellt, indem sie $C\alpha$-Atome jedes einer Vielzahl von Aminosäureresten identifiziert, indem sie die identifizierten $C\alpha$-Atome auf einem Umfang anordnet und indem sie Hauptketten und Seitenketten der Vielzahl von Aminosäureresten hinzufügt; und
eine Suchanweisungseinheit, die eine Sucheinheit veranlasst, nach einer stabilen Konformation des cyclischen Peptidmoleküls zu suchen, indem sie das erzeugte Modell als Ausgangskonformation des cyclischen Peptidmoleküls verwendet; und
eine Berechnungseinheit, die eine Diederwinkelverteilung auf der Grundlage von Backbone-Diederwinkeln berechnet, die bei der Suche nach der stabilen Konformation berechnet werden,
wobei die Suchanweisungseinheit die Sucheinheit veranlasst, nach der stabilen Konformation zu suchen, indem sie einen Suchbereich als Ausgangskonformation verwendet, wobei der Suchbereich durch die Diederwinkelverteilung angegeben wird und der Suchbereich richtige Antwortdaten einschließt, die den Diederwinkel jedes der Vielzahl von Aminosäureresten angeben.

**2.** Einrichtung zur Erzeugung einer Ausgangskonformation nach Anspruch 1, wobei
die Erzeugungseinheit die $C\alpha$-Atome in gleichen Abständen auf dem Umfang anordnet.

**3.** Einrichtung zur Erzeugung einer Ausgangskonformation nach Anspruch 1, wobei
die Erzeugungseinheit jedes der n $C\alpha$-Atome an einer Position mit den Koordinaten = [rsin (n-1) $\theta$x, rcos (n-1) $\theta$x ] anordnet, wobei der Abstand zwischen benachbarten $C\alpha$-Atomen a ist, r (a/2) $\times$ [sin ($\pi$/n)]$^{-1}$ist, $\theta$x x $\times$ 2$\pi$/n ist und x eine ganze Zahl von 1 bis n ist.

**4.** Einrichtung zur Erzeugung einer Ausgangskonformation nach Anspruch 1, wobei
die Erzeugungseinheit das Modell, das das zyklische Peptidmolekül darstellt, durch Umordnen einer Konformation nach dem Hinzufügen erzeugt.

**5.** Einrichtung zur Erzeugung einer Ausgangskonformation nach Anspruch 4, wobei
die Erzeugungseinheit die Konformation nach dem Hinzufügen neu anordnet, wobei die Anordnung der $C\alpha$-Atome unverändert bleibt.

**6.** Einrichtung zur Erzeugung einer Ausgangskonformation nach Anspruch 1, wobei
wobei die Berechnungseinheit die Diederwinkelverteilung in Farben ausgibt, die jeweils der Anzahl der durchgeführten Suchvorgänge nach der stabilen Konformation entsprechen.

**7.** Einrichtung zur Erzeugung einer Ausgangskonformation nach Anspruch 1, wobei
die Sucheinheit nach der stabilen Konformation durch ein verallgemeinertes Ensemble-Verfahren unter Verwendung des erzeugten Modells als Ausgangskonformation des cyclischen Peptidmoleküls sucht.

**8.** Verfahren zur Erzeugung einer Ausgangskonformation für einen Computer zur Ausführung eines Prozesses, umfassend:

Erzeugen eines Modells, das ein zyklisches Peptidmolekül darstellt, indem C$\alpha$-Atome jedes einer Vielzahl von Aminosäureresten identifiziert werden, indem die identifizierten C$\alpha$-Atome auf einem Umfang angeordnet werden und indem Hauptketten und Seitenketten der Vielzahl von Aminosäureresten hinzugefügt werden; und Veranlassen einer Sucheinheit, nach einer stabilen Konformation des cyclischen Peptidmoleküls zu suchen, indem das erzeugte Modell als Ausgangskonformation des cyclischen Peptidmoleküls verwendet wird; und Berechnen einer Diederwinkelverteilung auf der Grundlage von Rückgrat-Diederwinkeln, die bei der Suche nach der stabilen Konformation berechnet werden,

wobei das Veranlassen das Veranlassen der Sucheinheit umfasst, nach der stabilen Konformation zu suchen, indem ein Suchbereich als Ausgangskonformation verwendet wird, wobei der Suchbereich durch die Diederwinkelverteilung angegeben wird und der Suchbereich korrekte Antwortdaten einschließt, die den Diederwinkel jedes der mehreren Aminosäurereste angeben.

9. Programm zur Erzeugung einer Ausgangskonformation, das mindestens einen Computer veranlasst, einen Prozess auszuführen, wobei der Prozess Folgendes umfasst:

Erzeugen eines Modells, das ein zyklisches Peptidmolekül darstellt, indem C$\alpha$-Atome jedes einer Vielzahl von Aminosäureresten identifiziert werden, indem die identifizierten C$\alpha$-Atome auf einem Umfang angeordnet werden und indem Hauptketten und Seitenketten der Vielzahl von Aminosäureresten hinzugefügt werden; und Veranlassen einer Sucheinheit, nach einer stabilen Konformation des cyclischen Peptidmoleküls zu suchen, indem das erzeugte Modell als Ausgangskonformation des cyclischen Peptidmoleküls verwendet wird; und Berechnen einer Diederwinkelverteilung auf der Grundlage von Rückgrat-Diederwinkeln, die bei der Suche nach der stabilen Konformation berechnet werden,

wobei das Veranlassen das Veranlassen der Sucheinheit umfasst, nach der stabilen Konformation zu suchen, indem ein Suchbereich als Ausgangskonformation verwendet wird, wobei der Suchbereich durch die Diederwinkelverteilung angegeben wird und der Suchbereich korrekte Antwortdaten einschließt, die den Diederwinkel jedes der mehreren Aminosäurereste angeben.

## Revendications

1. Appareil de génération de conformation initiale, comprenant :

une unité de génération qui génère un modèle représentant une molécule de peptide cyclique en identifiant des atomes C$\alpha$ de chaque résidu parmi une pluralité de résidus d'acides aminés, en disposant les atomes C$\alpha$ identifiés sur une circonférence, et en ajoutant des chaînes principales et des chaînes latérales de la pluralité de résidus d'acides aminés ; et
une unité d'instruction de recherche qui amène une unité de recherche à rechercher une conformation stable de la molécule de peptide cyclique en utilisant le modèle généré pour servir de conformation initiale de la molécule de peptide cyclique ; et
une unité de calcul qui calcule une distribution d'angles dièdre sur la base d'angles dièdres de dorsale calculés lorsque la conformation stable est recherchée,
dans lequel l'unité d'instruction de recherche amène l'unité de recherche à rechercher la conformation stable en utilisant une plage de recherche pour servir de conformation initiale, la plage de recherche étant indiquée par la distribution d'angles dièdre, la plage de recherche incluant des données de réponses correctes qui indiquent l'angle dièdre de chaque résidu parmi la pluralité de résidus d'acides aminés.

2. Appareil de génération de conformation initiale selon la revendication 1, dans lequel l'unité de génération dispose les atomes C$\alpha$ à intervalles égaux sur la circonférence.

3. Appareil de génération de conformation initiale selon la revendication 1, dans lequel
l'unité de génération dispose chacun des n atomes C$\alpha$ au niveau d'une position de coordonnées = [rsin (n-1) $\theta$x, rcos (n-1) $\theta$x], une distance entre des atomes C$\alpha$ adjacents étant a, r étant (a/2) $\times$ [sin ($\pi$/n)]$^{-1}$, $\theta_x$ étant x $\times$ 2$\pi$/n et x étant un nombre entier de 1 à n.

4. Appareil de génération de conformation initiale selon la revendication 1, dans lequel
l'unité de génération génère le modèle représentant la molécule de peptide cyclique en redisposant une conformation après l'ajout.

**5.** Appareil de génération de conformation initiale selon la revendication 4, dans lequel l'unité de génération redispose la conformation après l'ajout en conservant la disposition des atomes Cα fixés.

**6.** Appareil de génération de conformation initiale selon la revendication 1, dans lequel l'unité de calcul sort la distribution d'angles dièdre dans des couleurs correspondant chacune au nombre de recherches de la conformation stable effectuées.

**7.** Appareil de génération de conformation initiale selon la revendication 1, dans lequel l'unité de recherche recherche la conformation stable par une méthode d'ensemble généralisé en utilisant le modèle généré pour servir de conformation initiale de la molécule de peptide cyclique.

**8.** Procédé de génération de conformation initiale pour un ordinateur pour exécuter un processus comprenant :

générer un modèle représentant une molécule de peptide cyclique en identifiant des atomes Cα de chaque résidu parmi une pluralité de résidus d'acides aminés, en disposant les atomes Cα identifiés sur une circonférence et en ajoutant des chaînes principales et des chaînes latérales de la pluralité de résidus d'acides aminés ; et amener une unité de recherche à rechercher une conformation stable de la molécule de peptide cyclique en utilisant le modèle généré pour servir de conformation initiale de la molécule de peptide cyclique ; et calculer une distribution d'angles dièdre sur la base d'angles dièdres de dorsale calculés lorsque la conformation stable est recherchée,
dans lequel le fait d'amener inclut le fait d'amener l'unité de recherche à rechercher la conformation stable en utilisant une plage de recherche pour servir de conformation initiale, la plage de recherche étant indiquée par la distribution d'angles dièdre, la plage de recherche incluant des données de réponses correctes qui indiquent l'angle dièdre de chaque résidu parmi la pluralité de résidus d'acides aminés.

**9.** Programme de génération de conformation initiale qui amène au moins un ordinateur à exécuter un processus, le processus comprenant :

générer un modèle représentant une molécule de peptide cyclique en identifiant des atomes Cα de chaque résidu parmi une pluralité de résidus d'acides aminés, en disposant les atomes Cα identifiés sur une circonférence et en ajoutant des chaînes principales et des chaînes latérales de la pluralité de résidus d'acides aminés ; et amener une unité de recherche à rechercher une conformation stable de la molécule de peptide cyclique en utilisant le modèle généré pour servir de conformation initiale de la molécule de peptide cyclique ; et calculer une distribution d'angles dièdre sur la base d'angles dièdres de dorsale calculés lorsque la conformation stable est recherchée,
dans lequel le fait d'amener inclut le fait d'amener l'unité de recherche à rechercher la conformation stable en utilisant une plage de recherche pour servir de conformation initiale, la plage de recherche étant indiquée par la distribution d'angles dièdre, la plage de recherche incluant des données de réponses correctes qui indiquent l'angle dièdre de chaque résidu parmi la pluralité de résidus d'acides aminés.

# FIG. 1

TERMINAL APPARATUS ─110

AMINO ACID RESIDUE SEQUENCE ACQUISITION UNIT ─111

CYCLIC PEPTIDE MOLECULE GENERATION UNIT ─112

DIHEDRAL ANGLE DISTRIBUTION CALCULATION UNIT ─113

STABLE CONFORMATION OUTPUT UNIT ─114

INITIAL CONFORMATION OF CYCLIC PEPTIDE MOLECULE

DIHEDRAL ANGLES

STABLE CONFORMATION OF CYCLIC PEPTIDE MOLECULE

SERVER APPARATUS ─120

STABLE CONFORMATION SEARCH UNIT ─121

# FIG. 2A

TERMINAL APPARATUS _110_

| PROCESSOR _201_ | MEMORY _202_ | AUXILIARY STORAGE DEVICE _203_ |

_207_

| I/F DEVICE _204_ | COMMUNICATION DEVICE _205_ | DRIVING DEVICE _206_ |

OPERATION DEVICE _211_ | OUTPUT DEVICE _212_ | RECORDING MEDIUM _213_

# FIG. 2B

_120_

SERVER APPARATUS

SERVER APPARATUS

SERVER APPARATUS

| PROCESSOR _221_ | MEMORY _222_ | AUXILIARY STORAGE DEVICE _223_ |

_227_

| I/F DEVICE _224_ | COMMUNICATION DEVICE _225_ | DRIVING DEVICE _226_ |

# FIG. 3

CYCLIC PEPTIDE MOLECULE GENERATION UNIT — 112

Phe1-Val2-Gly3-Gly4-Thr5-Ser6-Phe7-Asp8

a →

Cα ATOM ARRANGEMENT UNIT — 301

ADDITION UNIT — 302

CONFORMATIONAL RELAXATION UNIT — 303

# FIG. 4

# FIG. 5

510

520

# FIG. 6

# FIG. 7

# FIG. 8

# FIG. 9

```
         ┌─────────────────────────────────┐
         │  START OF STABLE CONFORMATION   │
         │        SEARCH PROCESSING        │
         └─────────────────────────────────┘
                         │
                         ▼
     ┌───────────────────────────────────────┐
     │  ACQUIRE AMINO ACID RESIDUE SEQUENCES │ ～ S901
     └───────────────────────────────────────┘
                         │
                         ▼
     ┌───────────────────────────────────────┐
     │  GENERATE INITIAL CONFORMATION OF CYCLIC │ ～ S902
     │           PEPTIDE MOLECULE            │
     └───────────────────────────────────────┘
                         │
                         ▼
     ┌───────────────────────────────────────┐
     │    SEARCH FOR STABLE CONFORMATION     │ ～ S903
     └───────────────────────────────────────┘
                         │
                         ▼
     ┌───────────────────────────────────────┐
     │  CALCULATE DIHEDRAL ANGLE DISTRIBUTION │ ～ S904
     └───────────────────────────────────────┘
                         │
                         ▼
     ┌───────────────────────────────────────┐
     │       OUTPUT STABLE CONFORMATION      │ ～ S905
     └───────────────────────────────────────┘
                         │
                         ▼
         ┌─────────────────────────────────┐
         │  START OF STABLE CONFORMATION   │
         │        SEARCH PROCESSING        │
         └─────────────────────────────────┘
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010159218 A
- JP 2020091518 A
- US 20180260517

**Non-patent literature cited in the description**

- **F. JIANG** ; **H. GENG**. *Computational Methods for Studying Conformational Behaviors of Cyclic Peptides*, 2019